# EUROPEAN PATENT SPECIFICATION

(11) **EP 3 426 237 B1**
(45) Date of publication and mention of the grant of the patent: **23.08.2023**
(21) Application number: 17716067.8
(22) Date of filing: 08.03.2017
(51) Int. Cl.: A61K 31/22, A61K 31/04, A61K 31/202, A61K 31/375, A61K 31/7068, A61K 31/7072, A61P 25/28, A61P 43/00

(54) **METHOD FOR TREATING BRAIN ATROPHY**
VERFAHREN ZUR BEHANDLUNG VON HIRNATROPHIE
PROCÉDÉ DE TRAITEMENT D'UNE ATROPHIE CÉRÉBRALE

(30) Priority: 08.03.2016 WO PCT/NL2016/050160
(43) Date of publication of application: 16.01.2019
(60) Divisional application: 21205862.2
(73) Proprietor: N.V. Nutricia, 2712 HM Zoetermeer (NL)
(72) Inventor: DE WILDE, Mattheus Cornelis, 3584 CT Utrecht (NL)
(74) Representative: Nederlandsch Octrooibureau
(86) International application number: PCT/NL2017/050141
(87) International publication number: WO 2017/155396

(56) References cited:
- WO-A1-02/088159
- WO-A1-2013/129914
- WO-A1-2013/149384
- WO-A1-2015/140545
- STEINBRENNER HOLGER ET AL: "Selenium homeostasis and antioxidant selenoproteins in brain: Implications for disorders in the central nervous system", ARCHIVES OF BIOCHEMISTRY AND BIOPHYSICS, vol. 536, no. 2, 13 March 2013 (2013-03-13), pages 152-157, XP028681014, ISSN: 0003-9861, DOI: 10.1016/J.ABB.2013.02.021
- "Abstracts", JOURNAL OF NEUROLOGICAL SCIENCES, ELSEVIER SCIENTIFIC PUBLISHING CO, AMSTERDAM, NL, vol. 229-230, 15 March 2005 (2005-03-15), pages 293-384, XP027710839, ISSN: 0022-510X [retrieved on 2005-03-15]

## Description

The invention is in the field of medical nutrition and more particularly relates to a composition for use in the treatment of a subject suffering from brain atrophy or brain shrinkage.

### BACKGROUND DESCRIPTION

Brain atrophy is a feature of the ageing brain, but also a common feature of many diseases affecting the brain, with the pattern and rate of progression of the atrophy being dependent on the disease involved. Brain atrophy or cerebral atrophy can be described as the loss of neurons and the connections in-between, causing shrinkage of the brain volume. Atrophy can be generalized, which means that all of the brain has shrunk, or it can be focal, affecting only a limited area of the brain and resulting in a decrease of the functions that area of the brain controls. Symptoms of significant brain atrophy include progressive cognitive impairment involving multiple cognitive functions, otherwise known as dementia, seizures and aphasia, which is the disruption in the understanding or production of language or both.

Several diseases can cause loss of synaptic connectivity and significant atrophy of the brain. Progressive neurodegenerative diseases of the brain, such as Alzheimer's disease, Parkinson's disease, Huntington's disease, corticobasal degeneration, posterior cortical atrophy, multiple system atrophy and progressive supranuclear palsy, cause increased rates of brain atrophy. They initially cause focal atrophy of specific brain regions and then they may gradually progress to generalized atrophy involving the entire brain, which ultimately results in death. Progressive brain atrophy provides a risk for neurodegenerative disease and cognitive and/or memory function impairment.

In Alzheimer's disease synapse loss and atrophy are the strongest structural correlates with cognitive impairment. From the very start of the disease process, before the disease is diagnosed, there is synaptic loss, reduced synaptic connectivity in specific brain areas, and a progressive atrophy. Some areas of the brain such as areas involved in short-term memory are generally more and earlier affected than others, and men lose more brain mass overall than women. Brain atrophy can be visualized using MRI, and generally does not affect all regions with the same intensity as shown by the neuroimaging.

Studies of improved nutrition in Alzheimer patients has shown improved neurocognitive outcomes in intervention groups, but whether increased nutritional intake in subjects suffering from brain atrophy and who are at risk of neurodegenerative diseases has not been adequately studied.

During the last decennium, uridine, choline and omega-3 fatty acids such as DHA have attracted attention as active components in treating cognitive dysfunction, often in context of age-associated neurodegenerative processes. These compounds have been found rate-limiting precursors for membrane phosphatide synthesis. WO 2012/125034 describes a composition comprising: i) one or more of uridine and cytidine, or salts, phosphates, acyl derivatives or esters thereof; ii) a lipid fraction comprising at least one of docosahexaenoic acid (22:6; DHA), eicosapentaenoic acid (20:5; EPA) and docosapentaenoic acid (22:5; DPA), or esters thereof, in which the lipid fraction comprises less than 2 weight% of linolenic acid (ALA), calculated on the weight of all fatty acids; iii) choline, or salts or esters thereof; for use in the prevention or treatment of neurotrauma, traumatic brain injury, cerebral palsy and spinal cord injury. WO 2013/129914 describes a composition comprising i) one or more of uridine and cytidine, or salts, phosphates, acyl derivatives or esters thereof; and ii) a lipid fraction comprising at least one of docosahexaenoic acid (22:6; DHA), eicosapentaenoic acid (20:5; EPA) and docosapentaenoic acid (22:5; DPA), or esters thereof for use in improving or preserving functional synaptic connectivity. In addition, WO 2015/140545 describes a combined therapy comprising the use of one or more omega -3 fatty acids and one or more B vitamins and for treating cognitive impairment by slowing its progression, such cognitive impairment disorders include Mild Cognitive Impairment (MCI) and Alzheimer's disease (AD).

There is a need to further investigate whether dietetic and nutritional intervention helps preventing and/or reducing brain atrophy.

### SUMMARY OF THE INVENTION

The inventors have observed that upon administering a combination or product comprising therapeutically effective amounts of (i) uridine monophosphate (UMP); (ii) docosahexaenoic acid (22:6; DHA) and eicosapentaenoic acid (20:5; EPA); (iii) vitamin E, vitamin C and selenium; (iv) vitamin B6, vitamin B9 and vitamin B12; (v) choline, a choline salt and/or choline ester, to a human subject the progression of brain atrophy can be reduced. Brain volume can be preserved using the composition of the invention, in particular in a subject at (high or increased) risk of a neurodegenerative disorder, which are characterized by brain atrophy, in a particular embodiment prodromal Alzheimer's disease.

The present invention concerns a composition for use in therapeutically preventing and/or slowing down brain atrophy in a human subject in need thereof, said composition comprising therapeutically effective amounts of (i) uridine monophosphate (UMP); (ii) docosahexaenoic acid (22:6; DHA) and eicosapentaenoic acid (20:5; EPA); (iii) vitamin E, vitamin C and selenium; (iv) vitamin B6, vitamin B9 and vitamin B12; (v) choline, a choline salt and/or choline ester.

The subject in need thereof is in particular a subject at (high or increased) risk of a neurodegenerative disorder such as Alzheimer's disease. In one embodiment, the human subject has suspected or confirmed brain atrophy or is at risk of neurodegeneration or neurodegenerative disease.

The present inventors surprisingly found, through clinical study, that administration of a composition containing therapeutically effective amounts of (i) uridine monophosphate (UMP); (ii) docosahexaenoic acid (22:6; DHA) and eicosapentaenoic acid (20:5; EPA); (iii) vitamin E, vitamin C and selenium; (iv) vitamin B6, vitamin B9 and vitamin B 12; (v) choline, a choline salt and/or choline ester, showed a significant reduction in hippocampus atrophy and a reduction in brain atrophy. Compliance and tolerability were very high and side effects were relatively low. The results of the clinical study are summarized in the examples section.

### LIST OF FIGURES

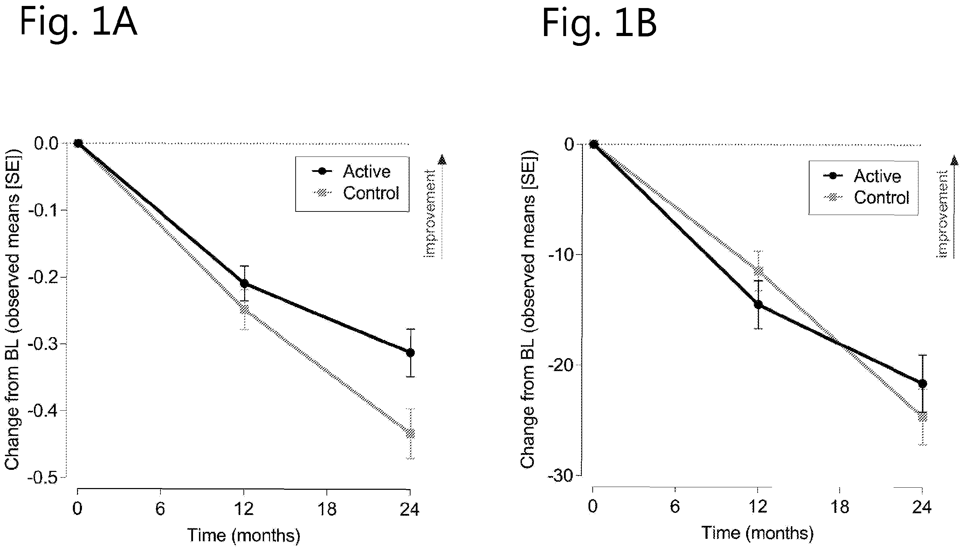

Figure 1A shows the changes in total hippocampal volume in cm³ over time of a group of subjects having brain atrophy randomized to either control treatment or nutritional supplementation with a composition according to the present invention. A significant slowing of hippocampus (left and right) atrophy was observed in the group that received the active composition.
Figure 1B shows the changes in total brain volume in cm³ over time of a group of subjects having brain atrophy randomized to either control treatment or nutritional supplementation with a composition according to the present invention. Slowing of brain atrophy was observed in the group that received the active composition.

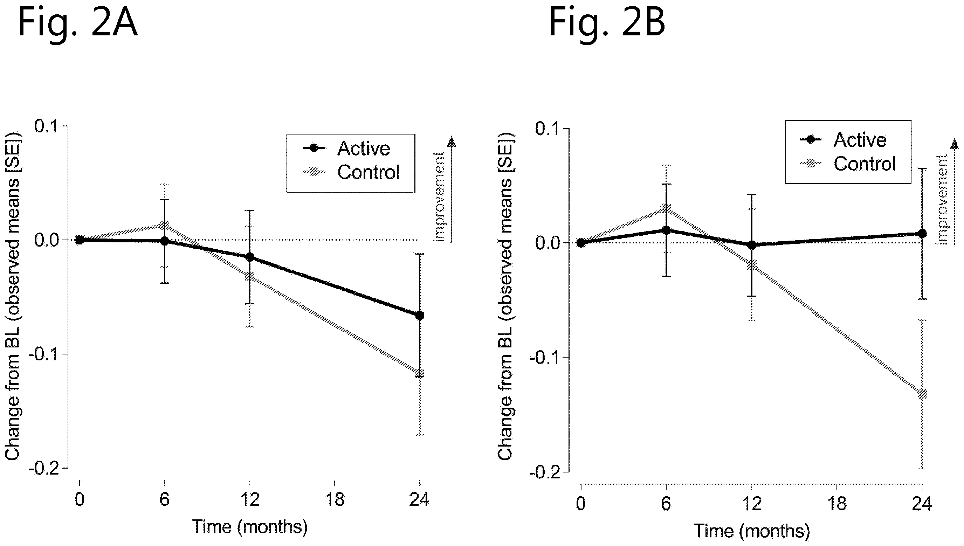

Figure 2 shows the changes in cognitive function over time of a group of subjects having brain atrophy randomized to either control treatment or nutritional supplementation with a composition according to the present invention. A clinical relevant trend of decreased cognitive function loss is observed in the group that received the active composition according to the invention. Figure 2A shows results for all randomised subjects, figure 2B shows the results for the per protocol population.

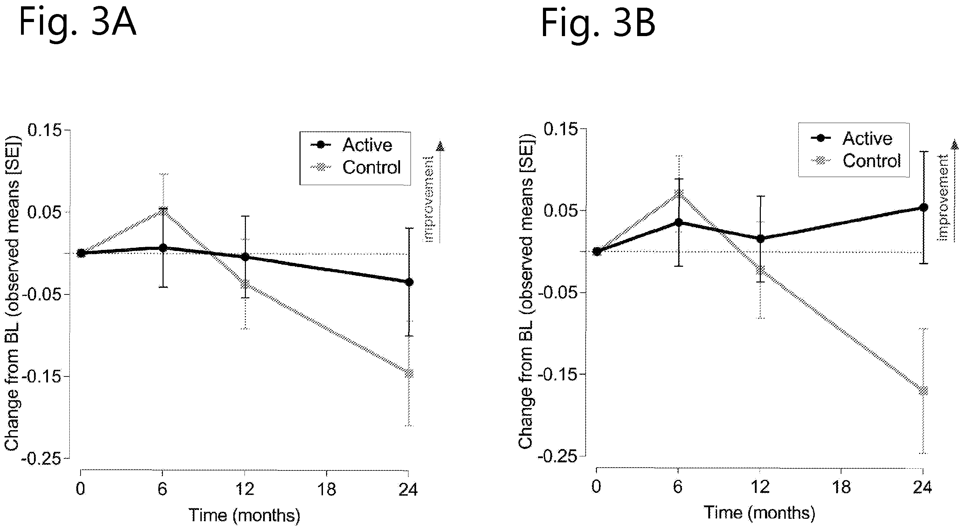

Figure 3 shows the change in memory function of the same groups of subjects having brain atrophy and being randomized to either control treatment or nutritional supplementation with a composition according to the present invention over time. Slowing down of the decline in memory function was observed for the group that received the active composition. Figure 3A shows results for all randomised subjects, figure 3B shows the results for the per protocol population.

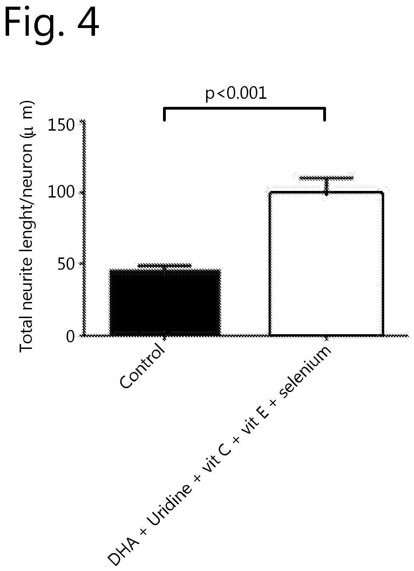

Figure 4 shows that a combination of DHA, uridine, Vitamin C, Vitamin E and selenium significantly (p<0.0001) improved neurite outgrowth (as measured as total neurite length per neuron) of PC12 cells *in vitro.*

### DETAILED DESCRIPTION OF THE INVENTION

Throughout this application, the following terminology and abbreviations may be used; Brain atrophy of cerebral atrophy is characterized by gradual loss of brain volume and loss of synapse connectivity. The extend of brain atrophy is generally determined using MRI wherein the volume of the brain and specific brain regions is determined. Hippocampus atrophy refers to atrophy in the medial temporal brain region where the hippocampi are located. Hippocampal atrophy is usually accompanied by deficits in declarative, episodic, spatial and contextual memory performance, with a reliable relationship between the size of the hippocampus and memory function performance. Hippocampal atrophy is a key feature of both hippocampal sclerosis (HS) and Alzheimer's disease (AD). In the context of the invention, brain atrophy and hippocampal atrophy are considered pathological or impaired conditions that require therapeutic treatment.

Synaptic connectivity refers to connections formed between different neurons allowing transmission of signals between said neurons. The synapse plays a role in the formation of memory. Synaptogenesis is the formation of new synapses and synaptic connections.

Several diseases are causally linked to the development of brain atrophy. Atrophy may be caused by Alzheimer's disease, Cerebral palsy, Senile dementia, fronto-temporal dementia, and vascular dementia, Pick's disease, Parkinson's disease, Huntington's disease, and other genetic disorders that cause build-up of toxic levels of proteins in neurons, malnutrition.

Medial temporal lobe atrophy (MTA) score refers to a visual score determined on coronal T1w MRI images through the hippocampus at the level of the anterior pons and assesses 3 features: 1) width of the choroid fissure, 2) width of the temporal horn of the lateral ventricle and 3) height of the hippocampus, resulting in a score of 0 to 4 depending on the degree of widening of the choroid fissure, enlargement of the temporal horn and atrophy of the hippocampus, wherein a score ≥ 2 is considered abnormal in people under the age of 75 years.

Mini mental state exam (MMSE) refers to a 30 point questionnaire to measure cognitive impairment such as in the diagnosis and longitudinal assessment of Alzheimer's disease. Any score greater than or equal to 24 points (out of 30) indicates a normal cognition. Below this, scores can indicate severe (≤9 points), moderate (10-18 points) or mild (19-23 points) cognitive impairment. The raw score may be corrected for educational attainment and age.

NTB test refers to a Neuropsychological Test Battery which comprises a combination of several validated, cognitive tests, yielding measures of a subjects performance in the cognitive field. The NTB tests were used to assess memory function and cognitive function.

In one aspect of the present invention, (i) - (v) are part of a composition according to the invention which is used as a pharmaceutical product comprising one or more pharmaceutically acceptable carrier materials.

In a preferred aspect of the present invention, (i) - (v) are part of a composition which is used as a nutritional product, for example as a nutritional supplement, e.g., as an additive to a normal diet, as a fortifier, to add to a normal diet, or as a complete nutrition. The nutritional product preferably comprises at least one component selected from the group of fats, proteins, and carbohydrates. It preferably comprises between 30 - 60 en% carbohydrates, between 30 and 60 en% fat, and between 1 and 20 en% protein. It is understood that a nutritional product differs from a pharmaceutical product by the presence of nutrients which provide nutrition to the subject to which the composition is administered, in particular the presence of protein, fat, digestible carbohydrates and dietary fibres. It is preferably a composition complying with food for special medical purposes requirements, preferably according to EC directive FSMP 1999/21/EC It may further contain ingredients such as minerals, vitamins, organic acids, and flavouring agents.

A first aspect of the invention provides a composition or combination for use in therapeutically preventing and/or reducing brain atrophy of human subjects having brain atrophy and/or at risk of neurodegenerative disease by feeding said subject with:
(i) therapeutically effective amounts ofuridine monophosphate (UMP);
(ii) docosahexaenoic acid (22:6; DHA) and eicosapentaenoic acid (20:5; EPA);
(iii) vitamin E, vitamin C and selenium;
(iv) vitamin B6, vitamin B9 and vitamin B 12;
(v) choline, a choline salt and/or choline ester,
preferably for use in the prevention or treatment (slowing down) of brain atrophy.

The subject is a human subject, preferably an adult, more preferably an elderly person, preferably at least 55 years of age.

In an embodiment of the invention the subject may be at risk of or suffering from prodromal Alzheimer's disease.

With 'preventing and/or treating' it is intended to mean that the brain volume decrease or the progression of brain atrophy is slowed down with the use of the composition according to the invention compared to a control group of treatment-naïve subjects suffering from the same condition but not given the composition of the invention.

According to another embodiment of the invention, the composition is provided as a nutritional product or nutritional supplement. The product of the invention is an enteral composition, intended for oral administration. It is preferably administered in liquid form. In one embodiment, the food product is a liquid composition containing between 50 and 250 kcal per 100 ml, more preferably between 75 and 125 kcal per 100 ml.

Preferably, the composition is enterally administered to the patient at least one time per day for a period of at least 12 weeks, preferably at least 26 weeks, more preferably at least 1 year or at least 2 years.

The use of the invention comprises administering the composition comprising the aforementioned ingredients(i)-(v), and as further outlined below, to a subject in need thereof.

### ω-3 LC-PUFAs

The composition or combination of the invention comprises therapeutically effective amounts of omega-3 long-chain polyunsaturated fatty acid (LC PUFA; having a chain length of 18 and more carbon atoms) docosahexaenoic acid (22:6; DHA) and eicosapentaenoic acid (20:5; EPA).. EPA is converted to DPA (ω-3), increasing subsequent conversion of DPA to DHA in the brain. Hence, the present composition or combination preferably also contains a significant amount of EPA, so to further stimulate *in vivo* DHA formation.

The LCPUFAs (DHA, EPA and/or DPA) are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition or combination comprises at least DHA in triglyceride form. Suitable ω-3 LCPUFA and/or DHA sources include tuna oil, (other) fish oils, DHA-rich alkyl esters, algae oil, egg yolk, or phospholipids enriched with ω-3 LCPUFA e.g. phosphatidylserine-DHA. Preferably, a composition according to the invention comprises fish oil providing the omega-3 LCPUFA(s). Another particularly suitable source for the omega-3 LCPUFA(s) is algae oil.

If EPA, DHA and EPA are present, the total daily dosage of DHA+EPA+DPA taken together is in the range of 0.25 - 5 g, preferably 0.5 - 5 g, more preferably 0.75 - 2.5 g, per 100 g of the composition, or in terms of daily dosage, in the range of 0.25 - 5 g, preferably 0.5 - 5 g, more preferably 0.75 - 2.5 g, per day. In a preferred embodiment, these amounts are based on the total sum of DHA and EPA. DHA is administered in therapeutic amounts, preferably in an amount of at least 0.5 g per 100 g, more preferably 0.5 - 5 g per 100 g, most preferably 0.75 - 2 g per 100 g of the composition, or in terms of daily dosage, in the range of at least 0.5 g per 100 g, more preferably 0.5 - 5 g per 100 g, most preferably 0.75 - 2 g per day.

In terms of the composition, combination or method, the proportion of ω-3 LCPUFA (more preferably DHA+EPA+DPA) of the total fatty acids in the composition is preferably 5 to 95 wt%, more preferably 10 to 80 wt%, most preferably 15 to 70 wt%, even more preferably 20 to 60 wt% of the total fatty acids. The present composition or combination preferably comprises 5 to 95 wt% DHA based on total fatty acids, preferably 10 to 75 wt% DHA based on total fatty acids, more preferably 10 to 60 wt%, even more preferably 10 - 50 wt%, more preferably 10 - 40 wt%, especially at least 20 wt% DHA, based on total fatty acids of the composition or combination. The present composition or combination preferably comprises 5 to 95 wt% EPA based on total fatty acids, preferably 5 to 75 wt% EPA, even more preferably 5 - 50 wt%, more preferably 5 - 25 wt%, most preferably 5 - 15 wt%, based on total fatty acids of the composition or combination.

In the method, combination or composition of the invention, the ratio of the weight of DHA to EPA is preferably larger than 1, more preferably 2:1 to 10:1, more preferably 2:1 to 5:1. The ratios take into account and optimize the balance between DHA and precursors thereof to ensure optimal effectiveness while maintaining low-volume formulations.

If arachidonic acid (AA) is present or administered, it concerns a very low amount of AA, expressed in terms of a DHA/AA weight ratio in the present composition, combination or method of at least 5, preferably at least 6. If AA is administered, it preferably amounts to less than 5 g per 100 ml liquid product and/or less than 300 mg per 100 g dry weight of the composition or combination.

### Uridine, cytidine and/or equivalents thereof

In one embodiment, cytidine, CMP, citicoline (CDP-choline) may also be applied in addition to UMP.

The present composition comprises UMP, as UMP is most efficiently being taken up by the body. Hence, inclusion of UMP in the present composition enables a high effectivity or efficacy at the lowest dosage and/or the administration of a low volume to the subject. Preferably at least 50 weight% of the uridine in the present method, combination and composition is provided by UMP, more preferably at least 75 weight%, most preferably at least 95 weight%. Doses administered are given as UMP. The amount of uracil sources can be calculated taking the molar equivalent to the UMP amount (molecular weight 324 Dalton).

The present method preferably comprises the administration of uridine (the cumulative amount of uridine, deoxyuridine, uridine phosphates, nucleobase uracil and acylated uridine derivatives) in an amount of 0.1-5 g, preferably 0.2-2.5 g, more preferably 0.25-1 g per 100 g of the composition, or in terms of daily dosage, in the range of 0.1-5 g, preferably 0.2-2.5 g, more preferably 0.25-1 g per day. In one embodiment, the present method, combination and compostion may comprise the administration a uridine source in a concentration of 0.1-5 g, preferably 0.2-2.5 g, more preferably 0.25-1 g per, calculated as UMP, per 100 kcal product. The terms product, composition and combination are used interchangeably.

### Vitamin C, selenium and vitamin E

The composition according to the invention comprises vitamin C, vitamin E and selenium.

Vitamin C includes functional equivalents thereof, and may be present in an amount in the range of 20 to 1000 mg, in particular in the range of 30 to 500 mg, more in particular in the range of 50 to 150 mg per 100 g of the composition, or in terms of daily dosage, in the range of 20 to 1000 mg, in particular in the range of 30 to 500 mg, more in particular in the range of 50 to150 mg per day.

Selenium may be present in an amount of 0.01 - 0.5 mg, preferably 0.02 - 0.1 mg per 100 g of the composition, or in terms of daily dosage, in the range of 0.01 - 0.5 mg, preferably 0.02 - 0.1 mg per day.

Vitamin E refers to compounds having vitamin E activity as known in the art, typically tocopherol and/or an equivalent thereof. Vitamin E may be present as tocopherol and tocopherol equivalents in an amount in the range of 10 to 200 mg, in particular in the range of 10 to 100 mg, more in particular in the range of 20 to 50 mg per 100 g of the composition, or in terms of daily dosage, in an amount in the range of 10 to 200 mg, in particular in the range of 10 to 100 mg, more in particular in the range of 20 to 50 mg per day. The term `tocopherol and equivalent thereof, as used in this description, comprises tocopherols (e.g. alpha- and gamma-), tocotrienols, pharmaceutical and/or nutritional acceptable derivatives thereof and any combination thereof. The above numbers are based on alpha-tocopherol equivalents (alpha-TE), as recognized in the art.

### Choline

The composition according to the present invention comprises therapeutically effective amounts of choline, a choline salt and/or choline ester. Herein, the term 'choline' shall be considered to encompass all these equivalents. Choline salts are preferred. The choline salt is preferably selected from choline chloride, choline bitartrate, or choline stearate. The choline ester is preferably selected from the group consisting of phosphatidylcholine and lyso-phosphatidylcholine. The present method preferably comprises the administration of more than 0.1 g choline per 100 g, preferably 0.1 to 1 g choline per 100 g, more preferably 0.2 to 0.5 g choline per 100 g of the composition or combination, or in terms of daily dosage, more than 0.1 g choline per 100 g, preferably 0.1 to 1 g choline per 100 g, more preferably 0.2 to 0.5 g choline per day. The above numbers are based on choline, the amounts of choline equivalents or sources can be calculated taking the molar equivalent to choline into account, based on the molar mass of 104 g/mol choline.

### B Vitamins

The composition according to the present invention comprises therapeutically effective amounts of vitamin B6 (pyridoxine, pyridoxal, or pyridoxamine, or pyridoxine hydrochloride), vitamin B9 (folic acid or folate), and vitamin B12 (cobalamins). Functional equivalents are encompassed within these terms. Vitamin B6 is preferably administered to provide an amount of 100 - 500 mcg, preferably 150 - 300 mcg, based on 100 g of the composition or per day. Vitamin B9 is preferably administered to provide an amount to provide 50 to 1000 µg, in particular in the range of 100 to 1000 µg, more in particular in the range of 200 to 800 µg per 100 g of the composition or per day. Vitamin B9 may be present as folate, which includes folic acid, folinic acid, methylated, methenylated and formylated forms of folates, their salts or esters (e.g. C1-6 alkyl ester), as well as their derivatives with one or more glutamic acid, and all in either reduced or oxidized form. Preferably, vitamin B9 is provided as folic acid. Vitamin B 12 is preferably administered to provide an amount in the range of 0.5 to 20 µg, in particular in the range of 1 to 10 µg per 100 g of the composition or per day. The term "vitamin B 12" incorporates all cobalbumin equivalents known in the art.

The composition may further comprise a digestible carbohydrate fraction, preferably including a source of lactose and a source of polysaccharide.

### EXAMPLES

For a more complete understanding of the present disclosure, reference is now made to the following examples taken in conjunction with the accompanying drawings.

### Example 1. clinical trial

A randomised, controlled, double-blind, parallel-group, multi-country clinical study was undertaken wherein 311 prodromal subjects were either treated for 2 years with a composition according to the invention (active composition, per 125 ml: 300 mg EPA, 1200 mg DHA, 106 mg phospholipids, 400 mg choline, 625 mg UMP, 40 mg vitamin E, 80 mg vitamin C, 60 mcg selenium, 3 mcg vitamin B12, 1 mg vitamin B6, 400 mcg folic acid) or received an iso-caloric control.

Inclusion criteria for prodromal patients were defined as follows:
1) suffering from episodic memory disorder, defined as -1 SD on 2 out of 8 tests (further explained below) of which at least memory test score is -1 SD.
2) Evidence for underlying Alzheimer's disease pathology
   - Medial temporal lobe atrophy ≥ 1 determined on MRI images
   - Cerebral spinal fluid measurement of : β-amyloid ratio < 1 or p-tau > 60 or t-tau > 350, or,
   - An abnormal FDG-PET compatible with Alzheimer's disease type of changes.
3) Age between 55-85 years
4) MMSE ≥ 24 (≥ 20 for subjects with ≤ 6 years of formal education)
5) Informed consent from both the subject as well as a caregiver.

A total of 311 subjects were randomized into either the iso-caloric (itt n=158) or the active composition (itt n=153) group. The subjects were followed-up for 24 months. The tests used for inclusion in the clinical trial are the following tests:
Memory
   - FCSRT - delayed free recall* ≤ 8
   - FCSRT free recall - learning ≤ 22
   - WMS-R story delayed recall (%) <_75%
   - WMS-R delayed recall of figures (%) ≤ 75%
Non-memory
   - TMT A ≥ 60
   - TMT B ≥ 150
   - Symbol Digit Substitution Test ≤ 35 (120 sec.)
   - Category Fluency ≤ 16 (60 sec.)
Abbreviations:
   FCRT: Free and Cued Selective reminding test, WMS: Wechsler Memory Scale, TMT: Trial Making Test

Figure 1A shows the changes in total hippocampal volume in cm³ over time. Significant slowing of left and right hippocampus atrophy was observed in the active group (left: p= 0.052; right: p=0.016, data not shown). A significant slowing of total hippocampus (left and right) atrophy was observed in the group that received the active composition, MM slope p=0.011 in the itt group comprising all randomised subjects (MM * 24 months p-0.008).

Figure 1B shows the changes in total brain volume in cm³ over time. Slowing of total brain atrophy was observed in the group that received the active composition (MM slope p=0.033, MM* 24 months p=0.416 and ANCOVA 24 months p=0.420) for the itt group comprising all randomised subjects.

Figure 2 shows the changes in cognitive function over time. To assess cognitive function Z-scores were calculated based on at least 4 out of 5 NTB tests relevant for cognitive function (CERAD immediate & delayed recall & recognition, Category Fluency, LDST). The calculation was performed for each of the NTB components by subtracting the baseline mean of the item (over the entire ITT population) and dividing by the baseline SD of the item (over the entire ITT population). The resultant z-scores were averaged to obtain the composite z-score. A clinical relevant trend of decreased cognitive function loss is observed in the group that received the active composition according to the invention. Figure 2A shows the itt group with all randomised subjects, figure 2B shows the per protocol analysed subjects.

Figure 3 shows the change in memory function of the same groups of subjects over time. The memory composite Z-scores were calculated based on the performance of a subject in 3 NTB components (CERAD immediate & delayed recall & recognition ). The calculation was performed for each of the NTB components by subtracting the baseline mean of the item (over the entire ITT population) and dividing by the baseline SD of the item (over the entire ITT population). The resultant z-scores were averaged to obtain the composite z-score. The memory domain Z-score was only calculated if all 3 NTB components were available. Slowing of the decline in memory function was observed for the group that received the active composition. Figure 3A shows the itt group with all randomised subjects, figure 3B shows the per protocol analysed subjects.

Treatment adherence during the study period was > 90% for both groups and the study protocol was generally well tolerated without severe adverse effects being reported in either treatment group.

Overall a clear benefit was observed for the group of subjects that were randomized into the group receiving the active composition according to the invention, with a slower development of hippocampal and total brain atrophy .

### Example 2. Effect of exposure of healthy rats to the composition of the invention on synapses.

The additional value of vitamin C, vitamin E and selenium according to the invention was assessed in healthy rats supplemented with uridine (as uridine-5'-monophosphate) and fish oil (FO), containing DHA and EPA.

Rats were randomized to four treatment groups and fed 1 of the 4 intervention diets for 6 weeks (see Table 1). On completion of dietary treatment, rats were sacrificed, and brain samples were analyzed for levels of phospholipids, synaptic proteins, and 2 enzymes that are involved in the synthesis of phospholipids, i.e. choline-phosphate cytidylyltransferase (PCYT1A) and choline/ethanolamine phosphotransferase (CEPT1).

**Table 1: Diets comprising different amounts of the antioxidants, fish oil and UMP.**

| | **Diet (grams/100 gram diet)** | | | |
|---|---|---|---|---|
| **Nutrient** | **Diet 1** | **Diet 2** | **Diet 3** | **Diet 4** |
| | **antioxidant low** | **antioxidant high** | **antioxidant low & fish oil+UMP** | **antioxidant high & FO+UMP** |
| Vitamin C | 0 | 0.160 | 0 | 0.160 |
| Vitamin E | 0.000385 | 0.160 | 0.000385 | 0.160 |
| Selenium | 0.000007 | 0.00012 | 0.000007 | 0.00012 |
| Fish oil | - | - | 3.2 | 3.2 |
| UMP | - | - | 1.0 | 1.0 |

Levels of total and individual phospholipids (except for phosphatidylinositol), levels of the pre- and post-synaptic proteins Synapsin-1 and PSD-95, as well as the levels of the enzymes PCYT1A and CEPT1, were all significantly increased by combined supplementation of fish oil plus uridine and antioxidants, and levels were not enhanced by supplementation of fish oil plus uridine without antioxidants. See table 2 for results.

**Table 2. Effects of specific dietary intervention with antioxidants, fish oil and UMP in rats on brain levels of phospholipids, synaptic proteins, and enzymes involved in phospholipids synthesis. Values are mean ± SEM. * P<0.05 vs. AOX low, One-way ANOVA post hoc comparison.**

| | **Dietary intervention** | | | |
|---|---|---|---|---|
| **Brain Levels** | **antioxidant low** | **antioxidant high** | **antioxidant low & fish oil+UMP** | **antioxidant high & fish oil +UMP** |
| Total phospholipids (nmol/mg protein) | 365.9±15.7 | 363.4±18.1 | 392.5±21.5 | 457.7±17.8^{∗} |
| PC (nmol/mg protein) | 136.6±3 | 135.9±3.1 | 148.6±4.04 | 166.9±4.7^{∗} |
| PE (nmol/mg protein) | 93.9±3.7 | 93.9±4.5 | 102.1±5.2 | 120.8±4.8^{∗} |
| PS (nmol/mg protein) | 30.5±1.4 | 30.5±1.4 | 33±2 | 39.2±1.7^{∗} |
| SM (nmol/mg protein) | 19.7±0.7 | 19.6±1 | 20.3±1.2 | 24±0.8^{∗} |
| PI (nmol/mg protein) | 17.7±1. 8 | 17.2±1.7 | 17.9±1.4 | 21.3±1.5 |
| | | | | |
| PSD-95 (% of AOX low values) | 100±1.8 | 107.3±3.8 | 115.9±5.2 | 132.9±5.9^{∗} |
| Synapsin-1 (% of AOX low values) | 100±1.8 | 106.6±3.1 | 113.1±6.3 | 136.6±6.1^{∗} |
| b-Tubulin control protein (% of AOX low values) | 100±2.7 | 95.9±3.7 | 95.1±3.4 | 108.4±5.6 |
| | | | | |
| Brain PCYT1A (ng/mL) | 9.5±0.1 | 9.9±1 | 9.9±0.1 | 12.4±0.2^{∗} |
| Brain CEPT1 (ng/mL) | 11.4±0.2 | 12.04±0.2 | 12.06±0.1 | 14.01±0.2^{∗} |

The results show that the antioxidants, vitamin C, vitamin E and selenium, in a diet containing phospholipid precursors exert a synergistic effect for the precursors' effects on increasing levels of both membrane phospholipids and synaptic proteins, the indirect indicators of neurite formation, synaptogenesis and synaptic connectivity. Moreover, levels of the enzymes involved in phospholipid synthesis were also increased when the combination was supplemented and not by supplementing either FO plus uridine or AOX alone.

### Example 3: in vitro study of the effect of a composition according to the invention on neuron outgrowth

PC12 pheochromocytoma cells were grown in DMEM (Gibco), supplemented with 10% fetal bovine serum (FBS), penicillin (100 units/ml), and streptomycin (100 µg/ml) (Gibco), under a humidified atmosphere with 95% air and 5% CO2 at 37°C. Cells were seeded in a 96 well plates at a density of2000 cells/well, and after 24 hours supplemented with medium containing 20ng/ml nerve growth factor (NGF). The cells were exposed to either a composition according to the invention or left unsupplemented (control). The composition the cells were supplemented with is indicated in table 3. Supplementation of cells was performed in triplicate. These conditions were compared to nonsupplemented cells. After supplementation for 2 days with these combinations, cells were stained using Calcein-AM stain (2ng/microliter) and the nuclei were counterstained using Hoechst stain (0.6 microgram/ml), in 100 µl culture medium per well and incubated for 20-30 min at37°C. Subsequently, FITC and DAPI images (25 images per well) were taken using an Arrayscan XTI high content imaging system. Neurite outgrowth was quantified using the Neuronal profiling Bioapplication algorithm. Finally, all data were statistically analyzed using Graphpad Prism. The present data show that a combination of DHA, uridine, Vitamin C, Vitamin E and selenium significantly (p<0.0001) improved neurite outgrowth (as measured as total neurite length per neuron) of PC12 cells in vitro (figure 4).

Brain functioning, ranging from perception and motor control to cognition, heavily depends on the structural connectivity between neurons that supports coordinated activity in neuronal networks. At the cellular level, the shape of a neuron (e.g. neurite length, and number of neurites and branches) is critical for its function as it determines the number of inputs the neuron can receive and how those inputs are processed. The results obtained with PC12 cells exposed to the composition according the invention is indicative of new neurite formation in response to the composition according to the invention..

**Table 3. PC12 cells were supplemented with a composition comprising the nutrient DHA, uridine, vitamin C, vitamin E and selenium for 2 days.**

| **Nutrient** | **Concentration (µM)** |
|---|---|
| DHA | 15 |
| Uridine | 75 |
| Vitamin C | 225 |
| Vitamin E | 60 |
| Selenium | 0.24 |

### Example 4. A liquid composition according to the invention, comprising per 125 mL serving:

| | |
|---|---|
| • 4.9 g fat | • 40 mg vitamin E (alpha-TE) |
| • 300 mg EPA | • 80 mg vitamin C |
| • 1200 mg DHA | • 60 µg selenium |
| • 106 mg phospholipids | • 3 µg vitamin B12 |
| • 400 mg choline | • 1 mg vitamin B6 |
| • 625 mg UMP | • 400 µg folic acid |

## Claims

1. A composition for use in therapeutically preventing and/or slowing down brain atrophy in a human subject in need thereof, said composition comprising therapeutically effective amounts of (i) uridine monophosphate (UMP); (ii) docosahexaenoic acid (22:6; DHA) and eicosapentaenoic acid (20:5; EPA); (iii) vitamin E, vitamin C and selenium; (iv) vitamin B6, vitamin B9 and vitamin B12; (v) choline, a choline salt and/or choline ester.

2. The composition for use according to claim 1, wherein the subject in need of therapeutically preventing and/or slowing down brain atrophy suffers from or is at increased risk of a neurodegenerative disease.

3. The composition for use according to claim 1 and 2, wherein the subject suffers from or is at risk of Alzheimer's disease.

4. The composition for use according to any one of the preceding claims, wherein DHA is administered in an amount of at least 0.5 g per 100 g, preferably 0.5-5 g per 100 g of the product or per day.

5. The composition for use according to any of the preceding claims, wherein selenium is administered in an amount of 0.01 - 0.5 mg, preferably 0.02 - 0.1 mg per 100 g of the composition or per day.

6. The composition for use according to any of the preceding claims, wherein vitamin C is administered in an amount of 20 to 1000 mg, in particular in the range of 30 to 500 mg, more in particular in the range of 50 to 150 mg per 100 g of the composition or per day.

7. A combination of therapeutically effective amounts of (i) uridine monophosphate (UMP); (ii) docosahexaenoic acid (22:6; DHA) and eicosapentaenoic acid (20:5; EPA); (iii) vitamin E, vitamin C and selenium; (iv) vitamin B6, vitamin B9 and vitamin B12; (v) choline, a choline salt and/or choline ester, for use in therapeutically preventing and/or slowing down brain atrophy in a human subject in need thereof.

## Patentansprüche

1. Zusammensetzung zum therapeutischen Vorbeugen und/oder Verlangsamen der Hirnatrophie bei einem Menschen, der dieses benötigt, wobei die besagte Zusammensetzung therapeutisch effektive Mengen an (i) Uridinmonophosphat (UMP); (ii) Docosahexaensäure (22:6; DHA) und Eicosapentaensäure (20:5; EPA); (iii) Vitamin E, Vitamin C und Selen; (iv) Vitamin B6, Vitamin B9 und Vitamin B12; (v) Cholin, einem Cholinsalz und/oder Cholinester umfasst.

2. Zusammensetzung zur Verwendung nach Anspruch 1, wobei der Patient, der das therapeutische Vorbeugen und/oder Verlangsamen der Hirnatrophie benötigt, an einer neurodegenerativen Krankheit leidet oder ein erhöhtes Risiko dafür hat.

3. Zusammensetzung zur Verwendung nach Anspruch 1 und 2, wobei der Patient an der Alzheimer-Krankheit leidet oder davon bedroht ist.

4. Zusammensetzung zur Verwendung nach einem der vorherigen Ansprüche, wobei DHA in einer Menge von mindestens 0,5 g pro 100 g, vorzugsweise 0,5 - 5 g pro 100 g des Produkts oder pro Tag verabreicht wird.

5. Zusammensetzung zur Verwendung nach einem der vorherigen Ansprüche, wobei Selen in einer Menge von 0,01 - 0,5 mg, vorzugsweise 0,02 - 0,1 mg pro 100 g der Zusammensetzung oder pro Tag verabreicht wird.

6. Zusammensetzung zur Verwendung nach einem der vorherigen Ansprüche, wobei Vitamin C in einer Menge von 20 bis 1000 mg, speziell im Bereich von 30 bis 500 mg, noch spezieller im Bereich von 50 bis 150 mg pro 100 g der Zusammensetzung oder pro Tag verabreicht wird.

7. Kombination von therapeutisch effektiven Mengen an (i) Uridinmonophosphat (UMP); (ii) Docosahexaensäure (22:6; DHA) und Eicosapentaensäure (20:5; EPA); (iii) Vitamin E, Vitamin C und Selen; (iv) Vitamin B6, Vitamin B9 und Vitamin B12; (v) Cholin, einem Cholinsalz und/oder Cholinester zum therapeutischen Vorbeugen und/oder Verlangsamen der Hirnatrophie bei einem Menschen, der dieses benötigt.

## Revendications

1. Composition destinée à être utilisée pour prévenir et/ou ralentir thérapeutiquement une atrophie cérébrale chez un sujet humain en ayant besoin, ladite composition comprenant des quantités thérapeutiquement efficaces de (i) uridine monophosphate (UMP) ; (ii) acide docosahexaénoïque (22:6; DHA) et acide eicosapentaénoïque (20:5; EPA) ; (iii) vitamine E, vitamine C et sélénium ; (iv) vitamine B6, vitamine B9 et vitamine B12 ; (v) de la choline, un sel de choline et/ou un ester de choline.

2. Composition destinée à être utilisée selon la revendication 1, où le sujet ayant besoin de prévenir et/ou ralentir thérapeutiquement l'atrophie cérébrale souffre ou présente un risque accru de développer une maladie neurodégénérative.

3. Composition destinée à être utilisée selon les revendications 1 et 2, où le sujet souffre ou présente un risque de développer la maladie d'Alzheimer.

4. Composition destinée à être utilisée selon l'une quelconque des revendications précédentes, où le DHA est administré en une quantité d'au moins 0,5 g pour 100 g, de préférence 0,5 à 5 g pour 100 g de produit ou par jour.

5. Composition destinée à être utilisée selon l'une quelconque des revendications précédentes, où le sélénium est administré en une quantité de 0,01 à 0,5 mg, de préférence 0,02 à 0,1 mg pour 100 g de la composition ou par jour.

6. Composition à être utilisée selon l'une quelconque des revendications précédentes, où la vitamine C est administrée en une quantité de 20 à 1000 mg, en particulier dans la plage de 30 à 500 mg, plus particulièrement dans la plage de 50 à 150 mg pour 100 g de la composition ou par jour.

7. Combinaison de quantités thérapeutiquement efficaces de (i) uridine monophosphate (UMP) ; (ii) acide docosahexaénoïque (22:6; DHA) et acide eicosapentaénoïque (20:5; EPA) ; (iii) vitamine E, vitamine C et sélénium ; (iv) vitamine B6, vitamine B9 et vitamine B12 ; (v) de la choline, un sel de choline et/ou un ester de choline, destinée à être utilisée thérapeutiquement dans la prévention et/ou le ralentissement de l'atrophie cérébrale chez un sujet humain en ayant besoin.
